# EUROPÄISCHE PATENTSCHRIFT

(11) **EP 3 384 323 B1**
(45) Veröffentlichungstag und Bekanntmachung des Hinweises auf die Patenterteilung: **26.02.2020**
(21) Anmeldenummer: 16754198.6
(22) Anmeldetag: 25.07.2016
(51) Int. Cl.: G01V 3/10, G01N 33/24

(54) **BODENSENSOR**
GROUND SENSOR
CAPTEUR DE SOL

(30) Priorität: 02.12.2015 AT 510392015
(43) Veröffentlichungstag der Anmeldung: 10.10.2018
(73) Patentinhaber: Geoprospectors GmbH, 2514 Traiskirchen (AT)
(72) Erfinder: PREGESBAUER, Michael, 2500 Baden (AT)
(74) Vertreter: Puchberger & Partner Patentanwälte
(86) Internationale Anmeldenummer: PCT/EP2016/067651
(87) Internationale Veröffentlichungsnummer: WO 2017/092885

(56) Entgegenhaltungen:
- EP-A2- 1 241 488
- DE-U1-202004 011 921
- US-A1- 2013 113 648
- US-A1- 2014 097 831
- US-B1- 7 443 154
- JEAN-MICHEL THOMASSIN ET AL: "Polymer/carbon based composites as electromagnetic interference (EMI) shielding materials", MATERIALS SCIENCE AND ENGINEERING: R: REPORTS, Bd. 74, Nr. 7, 1. Juli 2013 (2013-07-01), Seiten 211-232, XP055163356, ISSN: 0927-796X, DOI: 10.1016/j.mser.2013.06.001

## Beschreibung

Die Erfindung betrifft eine Vorrichtung zur Erfassung der Beschaffenheit eines Untergrunds, eine landwirtschaftliche Arbeitsmaschine mit einer derartigen Vorrichtung sowie ein Verfahren zum Betrieb einer derartigen landwirtschaftlichen Arbeitsmaschine.

Aus dem Stand der Technik im Bereich der Geophysik ist es bekannt, Bodensensoren einzusetzen, welche auf Basis elektromagnetischer Induktion (EMI) die Beschaffenheit eines Untergrunds detektieren. Dabei wird von einer elektromagnetischen Sendespule ein Primärfeld erzeugt, welches im Untergrund in Abhängigkeit von der Bodenbeschaffenheit ein elektromagnetisches Sekundärfeld induziert, welches durch eine oder mehrere Empfangsspulen im Oberflächenbereich aufgenommen und ausgewertet wird.

Aus den Signalen der Empfangsspulen ergeben sich Leitfähigkeitswerte, aus denen, je nach der Konfiguration und Empfindlichkeit der Spulen, die Beschaffenheit des Untergrunds, beispielsweise die Dichte, die Wassersättigung und die Bodenart, in einer Tiefe von wenigen cm bis mehreren Metern bestimmt werden kann.

Da die EMI Technologie im Gegensatz zu anderen geophysikalischen Methoden bei nahezu allen Untergrundsituationen einsetzbar ist und auch vergleichsweise kostengünstig in der Anschaffung ist, hat sie sich als Messmethode in der teilflächenspezifischen Landwirtschaft (Precision Farming) etabliert. Der Hauptanwendungsbereich etablierter Systeme ist es, Bodeninhomogenitäten in eigenen Messfahrten lateral zu erfassen und danach dem Landwirt in Form von Karten und Plänen zur Verfügung zu stellen.

Dabei werden zum Teil motorisierte Systeme eingesetzt, die auf einem zumeist eigens konstruierten Anhängesystem montiert werden und über die Untersuchungsfläche gezogen werden. Dabei muss ein großer Abstand des Bodensensors zum Zugfahrzeug eingehalten werden, um ein die Qualität minderndes Messrauschen oder sonstige Fehler bei der Datenerfassung möglichst zu verhindern.

Beim operativen Betrieb bekannter Bodensensoren findet die Datenerfassung unabhängig von Bewirtschaftungsfahrten des Landwirts statt, da der Betrieb des Systems und die Auswertung der Daten Fachpersonal erfordert. Von der Datenaufnahme bis zur Lieferung der Karten mit den ausgewerteten Bodendaten läuft somit ein komplexer Prozess ab, in den der Landwirt nicht eingebunden wird.

Herkömmliche Vorrichtungen sind beispielsweise aus der US 2013/113648 A1, der US 2014/0097831 A1, der DE 20 2004 011 921 U1, der US 7,443,154 B1 und der EP 1 241 488 A2 bekannt.

Aus der angeführten EP 1 241 488 A2 sind ein Gerät und ein Verfahren für die Analyse und die Kartierung von Boden und Gelände bekannt, wobei der Sensor in einem Kastenanhänger angeordnet ist, welcher hinter einem motorisierten Fahrzeug nachgezogen wird.

Aufgabe der Erfindung ist es, die Nachteile der bestehenden Systeme zu beheben und einen Bodensensor sowie ein Verfahren zum Betrieb eines Bodensensors zu realisieren, das es dem Landwirt ermöglicht, flexibel und unabhängig bereits während der landwirtschaftlichen Arbeitsfahrt Bodeninformationen zu erfassen und diese auch gleichzeitig oder anschließend für eine Steuerung landwirtschaftlicher Arbeitsmaschinen zu verwenden.

Diese und andere Aufgaben werden erfindungsgemäß durch eine landwirtschaftliche Arbeitsmaschine gemäß Anspruch 1 sowie ein Verfahren zum Betrieb der Arbeitsmaschine gemäß Anspruch 10 gelöst.

Indem die Sendespule und die Empfangsspulen erfindungsgemäß in einem Gehäuse angeordnet sind, welches elektromagnetische Strahlen abschirmendes Material umfasst, wird sichergestellt, dass die durch die Zugmaschine erzeugten Störsignale die Messung nicht oder nur unwesentlich beeinflussen, sodass erstmals ermöglicht wird, den Bodensensor während einer landwirtschaftlichen Arbeitsfahrt einzusetzen.

Durch die in einem Hohlraum im Gehäuse angeordneten Sende- und Empfangsspulen erfolgt in bekannter Weise eine Analyse der Bodenbeschaffenheit, wobei Leitfähigkeitswerte des Untergrunds bestimmt werden. Die ermittelten Leitfähigkeitswerte werden herangezogen, um mittels 1D Inversion die Mächtigkeit der ersten physikalisch trennbaren Bodenschicht zu bestimmen. Die 1D Inversion basiert auf einer Levenberg-Marquard Inversion bei der die Daten von mehreren, vorzugsweise 4 unabhängigen Empfangsspulen aufgrund des akkumulierten Response in Tiefenwerte invertiert werden. Um die Tiefe exakt bestimmen zu können ist die Kenntnis der Sensorposition und Orientierung während EMI Messung vorteilhaft, da der akkumulierte Response höhenabhängig ist.

Erfindungsgemäß kann vorgesehen sein, dass das Gehäuse einen elektrisch nicht leitfähigen Verbundstoff, vorzugsweise einen mehrlagigen Glasfaserverbundwerkstoff, umfasst. Das Gehäuse kann auch aus glasfaserverstärktem Kunststoff gefertigt sein. Dadurch wird sichergestellt, dass das Gehäuse robust genug ist, um den Anforderungen des landwirtschaftlichen Betriebs zu genügen. Erfindungsgemäß kann ebenfalls vorgesehen sein, dass das Gehäuse - mit Ausnahme des im Folgenden beschriebenen Vlieses - zur Gänze aus diesem nicht leitfähigen Verbundstoff besteht.

Erfindungsgemäß kann vorgesehen sein, dass das Gehäuse ein elektromagnetische Strahlen abschirmendes Vlies umfasst, welches vorzugsweise in der gesamten Gehäusewand außer im Bereich unterhalb der Spulen angeordnet ist. Dieses Vlies dient dazu, die elektromagnetischen Störsignale der Zugmaschine und anderer Störquellen abzuleiten, sodass die im Inneren des Gehäuse angeordneten Spulen durch die Störsignale nicht beeinflusst werden. Um die eigentlichen Messsignale nicht zu schwächen, wird das Vlies nicht im Bereich unterhalb der Spulen angeordnet.

Erfindungsgemäß kann vorgesehen sein, dass das Vlies carbonbeschichtetes Polyester umfasst und im Inneren der Gehäusewand, vorzugsweise zwischen den Lagen eines mehrlagigen Verbundstoffes angeordnet ist. Alternativ dazu kann das Vlies auch im Inneren des Gehäuses auf die Gehäusewand aufgebracht, beispielsweise aufgeklebt sein.

Erfindungsgemäß kann vorgesehen sein, dass das Vlies im Niederfrequenzbereich eine elektromagnetische Dämpfung von 70dB bis 90dB, vorzugsweise 80dB, sowie eine mechanische Reißfestigkeit in Längsrichtung von 200 N/mm bis 300 N/mm, vorzugsweise 260 N/mm aufweist. Der Begriff Niederfrequenz bezeichnet in diesem Zusammenhang eine Frequenz von unter 2 Hz.

Erfindungsgemäß kann vorgesehen sein, dass das Gehäuse mehrere, vorzugsweise sechs, Glasfilamentgewebelagen umfasst, wobei das Vlies zwischen den Glasfilamentgewebelagen angeordnet ist. Besonders bevorzugt ist eine Ausführung bei der das Gehäuse fünf innenliegende Glasfilamentgewebelagen mit einer Dichte von 280 g/m2 sowie eine außenliegende Glasfilamentgewebelage mit einer Dichte von 163 g/m2 umfasst, wobei das Vlies zwischen der zweiten und der dritten Lage angeordnet ist.

Erfindungsgemäß kann vorgesehen sein, dass das Vlies mit einer externen Erdung verbindbar ist. Zu diesem Zweck kann ein mit dem Vlies verbundener Erdungsanschluss auf der Außenseite des Gehäuses vorgesehen sein.

Die Gehäusewand des Gehäuses kann eine Dicke von unter 5 mm, vorzugsweise 4 mm, aufweisen. Durch den oben beschriebenen mehrlagigen Aufbau der Gehäusewand ergibt sich trotz der dünnen Gehäusewand eine ausreichende mechanische Stabilität des Gehäuses. Erfindungsgemäß kann vorgesehen sein, dass das Gehäuse hermetisch abgeschlossen und das Innere des Gehäuses gegen Spritzwasser sowie Verschmutzung geschützt ist.

Erfindungsgemäß kann vorgesehen sein, dass zumindest ein Distanzsensor zur Bestimmung des Abstandes der Vorrichtung zum Untergrund vorgesehen ist. Dies ermöglicht die ausreichende Genauigkeit bei der Tiefenberechnung auf Grundlage der durch die Empfangsspulen gemessenen Signale sicherzustellen. Der Distanzsensor kann insbesondere im unteren Bereich des Gehäuses angeordnet sein.

Erfindungsgemäß kann vorgesehen sein, dass die Vorrichtung zumindest einen Neigungssensor zur Bestimmung der Neigung der Vorrichtung relativ zum Untergrund umfasst.

Erfindungsgemäß kann zumindest ein Lokalisierungsmodul, vorzugsweise ein GPS-Modul, vorgesehen sein. Dadurch wird ermöglicht, die durch den Bodensensor ermittelten Messwerte in Abhängigkeit von der konkreten Position abzuspeichern und somit eine topologische Karte der Bodenbeschaffenheit zu erstellen. Das GPS-Modul kann im oder am Gehäuse selbst oder auch extern angeordnet sein.

Erfindungsgemäß kann vorgesehen sein, dass Mittel zum Anschluss einer externen Recheneinheit und/oder eines externen Terminals vorgesehen sind.

Die Erfindung umfasst weiters eine landwirtschaftliche Arbeitsmaschine, insbesondere eine Zugmaschine wie beispielsweise einen Traktor, umfassend einen erfindungsgemäßen Bodensensor sowie eine Recheneinheit zur Umrechnung der durch die Empfangsspule empfangenen Signale in elektrische Leitfähigkeitswerte und Bodenparameter, beispielsweise Dichte, Feuchtigkeit und Bodenart.

Erfindungsgemäß kann vorgesehen sein, dass die Recheneinheit getrennt vom Bodensensor vorgesehen ist, sodass der Bodensensor entsprechende, insbesondere auch drahtlose Schnittstellen zur Übermittlung der gemessenen Signale der Empfangsspulen an die Recheneinheit aufweist.

Erfindungsgemäß kann vorgesehen sein, dass ein Terminal zur Ansteuerung des Bodensensors vorgesehen ist. Das Terminal kann insbesondere an der Arbeitsmaschine, beispielsweise in der Fahrerkabine, angeordnet sein. Auch der oben genannte GPS-Sensor kann erfindungsgemäß an der Arbeitsmaschine angeordnet und mit der Recheneinheit und/oder dem Terminal verbunden sein.

Erfindungsgemäß kann vorgesehen sein, dass der Bodensensor an einem Fronthubwerk der Arbeitsmaschine montiert ist.

Weiters kann erfindungsgemäß vorgesehen sein, dass an der Arbeitsmaschine Steuerausgänge zur Ansteuerung externer Bodenbearbeitungsgeräte vorgesehen sind. Die Steuerausgänge können vorzugsweise im Heckbereich der Arbeitsmaschine angeordnet sein, sodass eine unmittelbare Ansteuerung von im Heckbereich der Arbeitsmaschine angeordneten Bodenbearbeitungsgeräten auf Grundlage der während einer Arbeitsfahrt durch den Bodensensor detektierten Bodenbeschaffenheit ermöglicht wird.

Die Erfindung erstreckt sich weiters auf ein Verfahren zum Betrieb einer erfindungsgemäßen landwirtschaftlichen Arbeitsmaschine, wobei während einer Arbeitsfahrt der Arbeitsmaschine die Bodenparameter des aktuell zu bearbeitenden Bodenabschnittes detektiert werden. Dies ermöglicht erstmals, dass der Landwirt bereits während einer Arbeitsfahrt, beispielsweise während der Aussaat, die Bodenbeschaffenheit aufnimmt und somit kein separater Arbeitsschritt zur Untersuchung der Bodenbeschaffenheit erforderlich wird. Die Verwendung vorab aufgenommener Karten erübrigt sich somit für den Landwirt.

Erfindungsgemäß kann vorgesehen sein, dass zur Kalibrierung des Bodensensors zunächst eine einmalige statische Messung ohne Arbeitsmaschine durchgeführt und auf Grundlage des in der statischen Messung detektierten Hintergrundrauschens ein statischer Offset bestimmt wird, und in Folge die während der Arbeitsfahrten detektierten Signale um diesen statischen Offset korrigiert werden. Bei dem Hintergrundrauschen kann es sich insbesondere um weißes Rauschen handeln, welches sich über den gesamten betrachteten Frequenzbereich erstreckt und eine Amplitude des Leitwerts im Bereich von etwa 0,2 mS - 0,4 mS aufweist.

Dadurch wird erreicht, dass der durch die Arbeitsmaschine erzeugte statische Offset herausgefiltert werden kann. Dieser statische Offset ist jener Versatz, den die gemessenen Daten durch die laufende Arbeitsmaschine erfahren, indem der Bodensensor an der Arbeitsmaschine montiert ist. Der statische Offset wird erfindungsgemäß bestimmt, indem eine statische Messung in derselben Position und Orientierung einmal mit und einmal ohne die Arbeitsmaschine durchgeführt wird. Die Differenz der beiden Messungen ist der statische Offset; dieser wird einmalig für eine Arbeitsmaschine bestimmt.

Erfindungsgemäß kann vorgesehen sein, dass während der Arbeitsfahrten die detektierten Signale durch einen Filter, vorzugsweise einen Hampel-Filter gefiltert werden, um Messfehler und Ausreißer zu beseitigen. Statt des Hampel-Filters kann auch jeder andere Filter eingesetzt werden, der geeignet ist, Ausreißer in den aufgenommenen Messwerten zu detektieren und zu entfernen.

Erfindungsgemäß kann vorgesehen sein, dass die während der Arbeitsfahrten detektierten Signale durch einen adaptiven Tiefpass-Filter gefiltert werden, wobei die Parameter des Tiefpass-Filters während der Arbeitsfahrt anpassbar sind. Dadurch wird erreicht, dass neben dem oben genannten niederfrequenten Hintergrundrauschen auch hochfrequente Signalanteile gefiltert werden können. Die Filter können insbesondere als Software-Filter implementiert sein.

Die Grenzfrequenz des adaptiven Tiefpass-Filters kann vorab in einer Initialisierungsphase festgelegt werden und in Folge während einer Arbeitsfahrt bei Überschreitung eines Grenzwerts der Amplitude der gemessenen Signale angepasst werden. Dies bringt den Vorteil, dass der Filter auf die sich ändernden Bedingungen bei der Messung angepasst werden kann. Die Detektierung der sich ändernden Bedingungen kann beispielsweise dadurch erfolgen, dass die Amplitude der gemessenen Signale vor dem Filter über einen großen Frequenzbereich bestimmt wird, und die Grenzfrequenz des adaptiven Filters kontinuierlich auf jenen Wert gesetzt wird, bei dem die Amplitude überdurchschnittlich stark ansteigt, beispielsweise auf das 5- bis 10-fache des Wertes bei niedrigen Frequenzen.

Erfindungsgemäß kann vorgesehen sein, dass der Bodensensor während der Arbeitsfahrten von der Arbeitsmaschine in einer Geschwindigkeit von vorzugsweise maximal 15 km/h über den zu untersuchenden Untergrund bewegt wird, wobei die detektierte Beschaffenheit des Untergrunds, beispielsweise die Bodenart, unmittelbar zur Steuerung von im Heckbereich der Arbeitsmaschine montierten Bodenbearbeitungsgeräten verwendet wird. Dies bringt den erfindungsgemäßen Vorteil, dass der Landwirt bereits während der Bestellung des Feldes die Bodenbearbeitung auf die Beschaffenheit des Bodens einstellen kann.

Erfindungsgemäß laufen sämtliche Prozesse der Bodenerfassung automatisch ab, sodass der Landwirt die Datenerfassung nur starten muss. Die Kombination aus Software, Datenerfassungshardware und Montageort ermöglichen dem Landwirt eine betriebsbegleitende Anwendung. Sämtliche durch den Bodensensor erfassten Daten und berechneten Informationen stehen am Steuerausgang innerhalb der Bearbeitungsgeschwindigkeit des Schleppers zu Verfügung, wobei eine maximale Geschwindigkeit bestimmt wird, bei der die Länge der Arbeitsmaschine ausreichend ist, um die berechneten Daten am Steuerausgang in Echtzeit zur Verfügung zu stellen.

Erfindungsgemäß kann vorgesehen sein, dass während der Arbeitsfahrt eine Warnmeldung ausgegeben wird, sofern die Umweltbedingungen eine Erfassung der Beschaffenheit des Untergrunds nicht zulassen.

Erfindungsgemäß kann vorgesehen sein, dass die Beschaffenheit des Untergrunds in einer ersten Arbeitsfahrt vorab, beispielsweise während der Aussaat, aufgenommen und in einer Datenbank gespeichert wird und in einer späteren Arbeitsfahrt zur Bodenbearbeitung eingesetzt wird.

Weitere erfindungsgemäße Merkmale ergeben sich aus den Patentansprüchen, der Figurenbeschreibung und den Zeichnungen.

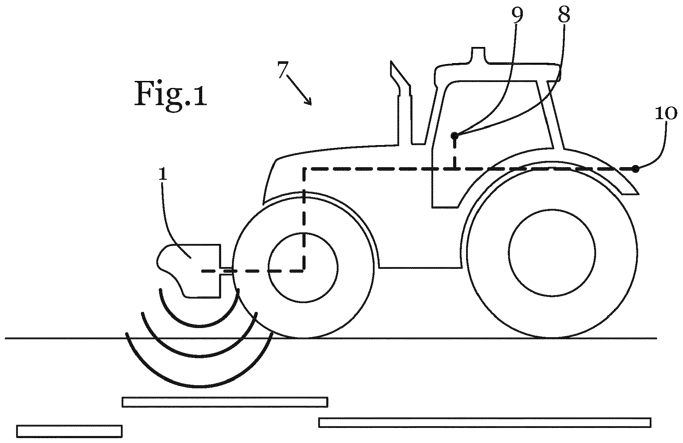

Fig. 1 zeigt eine schematische Darstellung einer Arbeitsmaschine 7 mit einem am Fronthubwerk montierten Bodensensor 1. Die vom Bodensensor 1 gemessenen Signale werden an eine Recheneinheit 8 in der Arbeitsmaschine 7 übermittelt und dort ausgewertet, um die Bodenbeschaffenheit zu detektieren. Dabei befindet sich die Arbeitsmaschine 7 in Bewegung, sodass kontinuierlich ein Bereich des Untergrunds analysiert werden kann.

Im Bereich der Recheneinheit 8 befindet sich auch ein Terminal 9 zur Steuerung des Bodensensors 1. Von der Recheineinheit 8 bzw. dem Terminal 9 führen Leitungen zu einem Steuerausgang 10 am Heck der Arbeitsmaschine 7, der verwendet werden kann, um nicht dargestellte Bodenbearbeitungsgeräte unter Bezug auf die ermittelte Bodenbeschaffenheit zu steuern. Am Heck der Arbeitsmaschine ist weiters ein mit der Recheneinheit 8 verbundener nicht dargestellter GPS-Empfänger angeordnet.

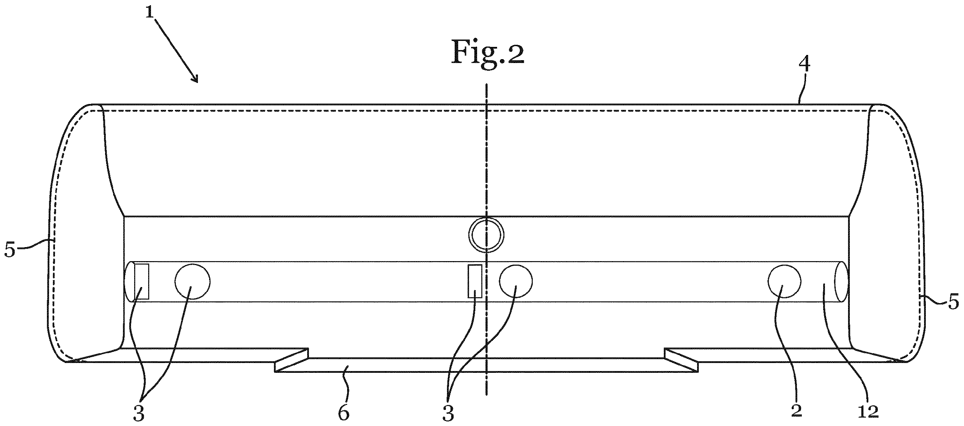

Fig. 2 zeigt eine schematische Darstellung des erfindungsgemäßen Bodensensors 1 in einer Ansicht von oben. Dieser umfasst ein Gehäuse 4 aus einem nicht leitfähigen Material mit einer Gehäusewand, in die ein schirmendes, elektrisch leitfähiges Vlies 5 eingearbeitet ist, welches über einen nicht dargestellten Erdungsanschluss an die Erdung der Arbeitsmaschine 7 angeschlossen werden kann. Die Gehäusewand umfasst mehrere Lagen eines Verbundmaterials aus Glasfilamentgewebe, und das Vlies 5 ist zwischen den einzelnen Lagen des Verbundmaterials eingearbeitet.

Im Inneren des Gehäuses 4 befindet sich ein Hohlraum 12, in dem eine Sendespule 12 sowie vier unterschiedlich orientierte Empfangsspulen 3 angeordnet sind. Das Vlies 5 ist im Bereich unterhalb der Spulen 2, 3 ausgespart, um das Senden und Empfangen der elektromagnetischen Signale nicht zu stören. Zur Befestigung des Bodensensors 1 am Fronthubwerk der Arbeitsmaschine 7 ist eine Montagevorrichtung 6 vorgesehen. Zur Stabilisierung des Gehäuses 4 befinden sich weiters im Inneren des Gehäuses die in dieser Darstellung nicht gezeigten Klammern 11, welche Ausnehmungen zur Einbringung der Spulen 2, 3 aufweisen.

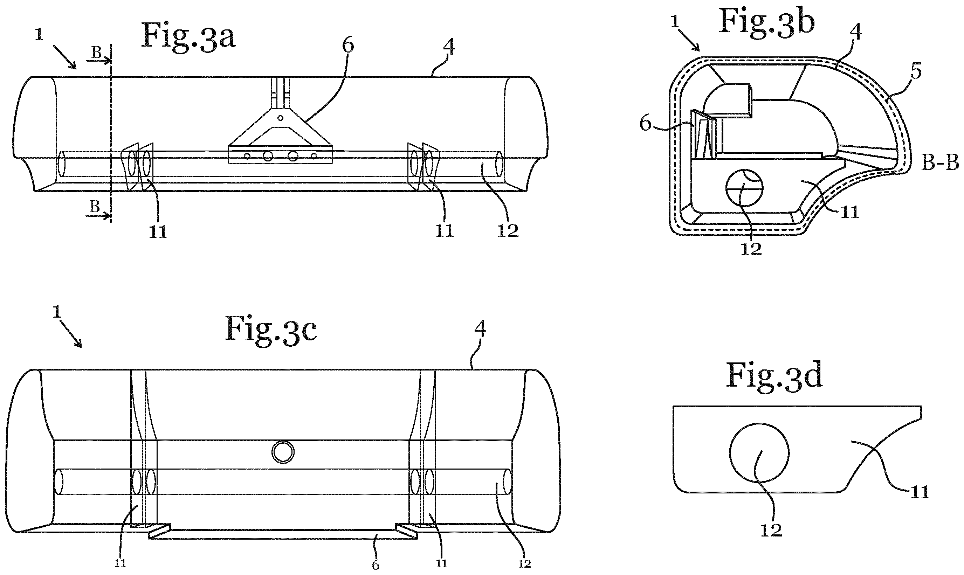

Fig. 3a - 3c zeigen weitere schematische Ansichten des erfindungsgemäßen Bodensensors 1. Fig. 3a zeigt die Ansicht des Bodensensors 1 von vorne, Fig. 3b zeigt den Bodensensor 1 von der Seite, und Fig. 3c zeigt den Bodensensor 1 von oben. In diesen schematischen Darstellungen sind die Spulen 2, 3 sowie die Distanzsensoren aus Gründen der Übersichtlichkeit nicht dargestellt. In der Schnittdarstellung gemäß Linie B-B in Fig. 3b ist die spezielle Formgebung des Gehäuses 4 sichtbar, welches am vorderen unteren Bereich eine ausgeprägte konkave Einbuchtung aufweist. Dies verhindert bei der Bewegung des Bodensensors 1 über den Untergrund, dass sich Hindernisse wie Steine oder Erdklumpen unter dem Bodensensor 1 verkanten und eine Messung behindern. Außerdem ist in Fig. 3b die Positionierung des Vlieses 5 schematisch dargestellt, welches innerhalb der Gehäusewand angeordnet ist. In Fig. 3a ist auch die Montagevorrichtung 6 schematisch dargestellt, welche sich an der Rückseite des Bodensensors 1 befindet.

Fig. 3d zeigt eine Ansicht einer Klammer 11, die zur Stabilisierung im Inneren des Gehäuses 4 angeordnet ist und einen kreisförmigen Hohlraum 12 zur Aufnahme der Spulen 2, 3 aufweist.

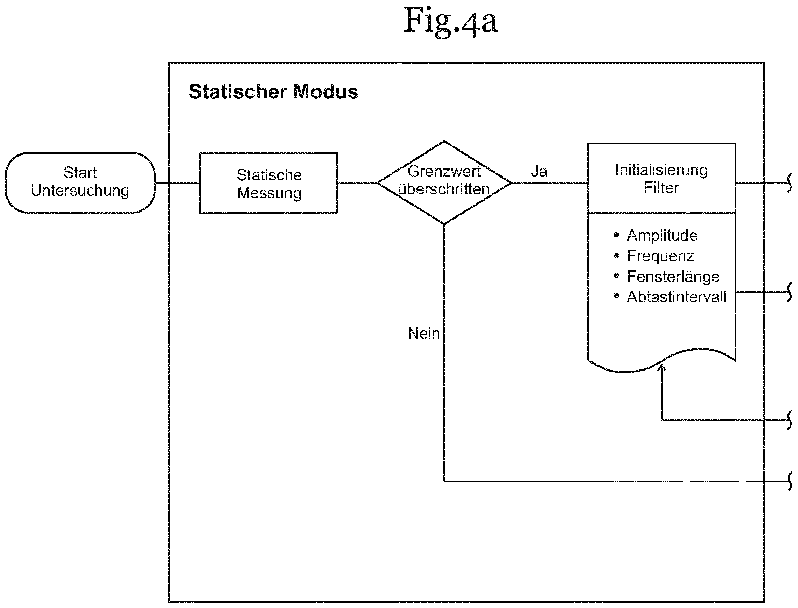

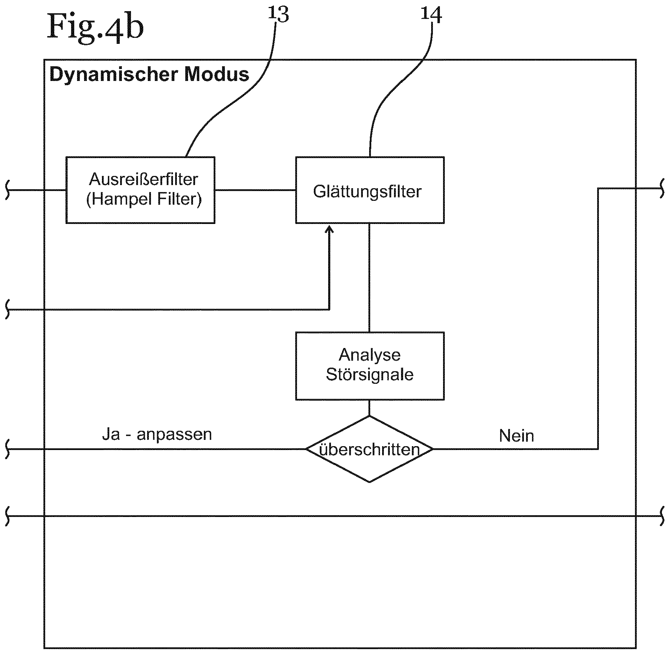

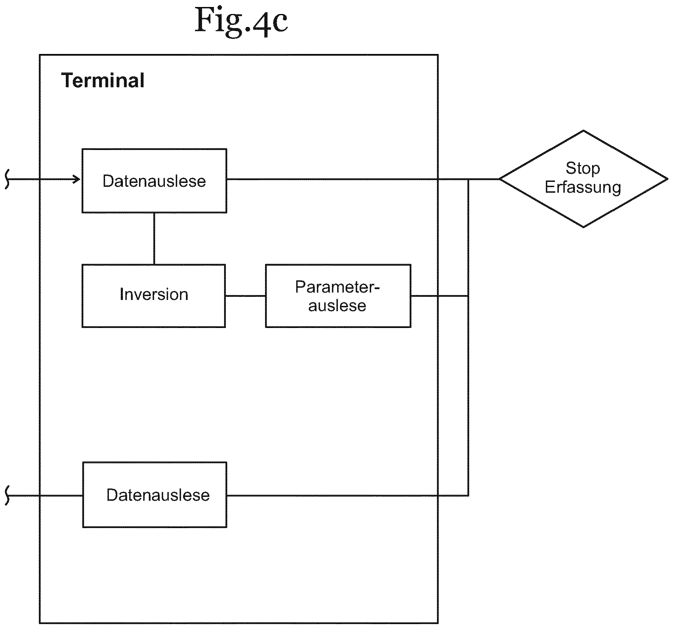

Fig. 4a - 4c zeigt ein schematisches Ablaufdiagramm einer Ausführungsform des erfindungsgemäßen Verfahrens zum Betrieb einer landwirtschaftlichen Arbeitsmaschine mit einem erfindungsgemäßen Bodensensor.

Vor der eigentlichen Messung wird eine statische Initialisierung (Static Mode) der Anlage durchgeführt. Dabei werden zwei Filter initialisiert, nämlich einerseits ein Hampel-Filter 13 zur Filterung von Ausreißern, und andererseits ein Glättungsfilter 14, der als Tiefpass augeführt ist. Während der Messphase (Dynamic Mode) werden kontinuierlich die gemessenen Leitfähigkeits-Werte und die Störsignale analysiert (Analyse noise) und die Parameter des Glättungsfilters gegebenenfalls über eine Rückkoplungsschleife angepasst.

Am Terminal bzw. der Recheineinheit erfolgt schließlich die Berechnung der 1D-Inversion zur Bestimmung der Bodenparameter aus den von den vier Spulen empfangenen Leitfähigkeitswerten.

## Patentansprüche

1. Landwirtschaftliche Arbeitsmaschine (7), insbesondere Zugmaschine, umfassend einen Bodensensor (1), zur Erfassung der Beschaffenheit eines Untergrunds, wobei der Bodensensor (1) zumindest eine Sendespule (2) und zumindest eine, vorzugsweise vier, Empfangsspulen (3), umfasst, wobei die Sendespule (2) zur Erzeugung eines elektromagnetischen Primärfelds und die Empfangsspule (3) zum Empfangen des im Untergrund durch das Primärfeld induzierten elektromagnetischen Sekundärfelds eingerichtet ist, wobei die Sendespule (2) und die Empfangsspule (3) in einem Gehäuse (4) angeordnet sind, welches elektromagnetische Strahlen abschirmendes Material umfasst, **dadurch gekennzeichnet, dass** der Bodensensor (1) an einem Fronthubwerk der Arbeitsmaschine (7) montiert ist.

2. Landwirtschaftliche Arbeitsmaschine (7) nach Anspruch 1, **dadurch gekennzeichnet, dass** das Gehäuse (4) des Bodensensors einen elektrisch nicht leitfähigen Verbundstoff, vorzugsweise einen mehrlagigen Glasfaserverbundwerkstoff, umfasst.

3. Landwirtschaftliche Arbeitsmaschine (7) nach Anspruch 1 oder 2, **dadurch gekennzeichnet, dass** das Gehäuse (4) des Bodensensors ein elektromagnetische Strahlen abschirmendes Vlies (5) umfasst, welches vorzugsweise in der gesamten Gehäusewand außer im Bereich unterhalb der Spulen (2, 3) angeordnet ist.

4. Landwirtschaftliche Arbeitsmaschine (7) nach Anspruch 3, **dadurch gekennzeichnet, dass** eine mit dem Vlies (5) elektrisch verbundene Erdungsleitung vorgesehen ist, um das Vlies (5) mit einer externen Erdung einer Arbeitsmaschine zu verbinden.

5. Landwirtschaftliche Arbeitsmaschine (7) nach einem der Ansprüche 1 bis 4, **dadurch gekennzeichnet, dass** an dem Bodensensor zumindest ein Distanzsensor zur Bestimmung des Abstandes der Vorrichtung zum Untergrund vorgesehen ist.

6. Landwirtschaftliche Arbeitsmaschine (7) nach einem der Ansprüche 1 bis 5, **dadurch gekennzeichnet, dass** an dem Bodensensor zumindest ein Neigungssensor zur Bestimmung der Neigung der Vorrichtung relativ zum Untergrund vorgesehen.

7. Landwirtschaftliche Arbeitsmaschine (7) nach einem der Ansprüche 1 bis 6, **dadurch gekennzeichnet, dass** an dem Bodensensor zumindest ein Lokalisierungsmodul, vorzugsweise ein GPS-Modul, vorgesehen ist.

8. Landwirtschaftliche Arbeitsmaschine (7) nach einem der Ansprüche 1 bis 7, **dadurch gekennzeichnet, dass** eine Recheneinheit (8) zur Umrechnung der durch die Empfangsspule (3) empfangenen Signale in elektrische Leitfähigkeitswerte und Bodenparameter, beispielsweise Dichte, Feuchtigkeit und Bodenart, vorgesehen ist.

9. Landwirtschaftliche Arbeitsmaschine nach einem der Ansprüche 1 bis 8, **dadurch gekennzeichnet, dass** an der Arbeitsmaschine (7) Steuerausgänge (10) zur Ansteuerung externer Bodenbearbeitungsgeräte vorgesehen sind, wobei die Steuerausgänge (10) vorzugsweise im Heckbereich der Arbeitsmaschine angeordnet sind, sodass eine unmittelbare Ansteuerung von im Heckbereich der Arbeitsmaschine angeordneten Bodenbearbeitungsgeräten auf Grundlage der während einer Arbeitsfahrt durch den Bodensensor (1) detektierten Bodenbeschaffenheit ermöglicht wird.

10. Verfahren zum Betrieb einer landwirtschaftlichen Arbeitsmaschine nach einem der Ansprüche 1 bis 9, **dadurch gekennzeichnet, dass** während einer Arbeitsfahrt der Arbeitsmaschine die Bodenparameter des aktuell zu bearbeitenden Bodenabschnittes detektiert werden.

11. Verfahren nach Anspruch 10, **dadurch gekennzeichnet, dass** zur Kalibrierung des Bodensensors (1) zunächst eine einmalige statische Messung ohne Arbeitsmaschine durchgeführt und auf Grundlage des in der statischen Messung detektierten Hintergrundrauschens ein statischer Offset bestimmt wird, und in Folge die während der Arbeitsfahrten detektierten Signale um diesen statischen Offset korrigiert werden.

12. Verfahren nach Anspruch 10 oder 11, **dadurch gekennzeichnet, dass** die während der Arbeitsfahrten detektierten Signale durch einen adaptiven Tiefpass-Filter gefiltert werden, wobei die Parameter des Tiefpass-Filters während der Arbeitsfahrt anpassbar sind.

13. Verfahren nach Anspruch 12, **dadurch gekennzeichnet, dass** die Grenzfrequenz des Tiefpass-Filters in einer Initialisierungsphase festgelegt wird und während einer Arbeitsfahrt bei Überschreitung eines Grenzwerts der Amplitude der gemessenen Signale angepasst wird.

14. Verfahren nach einem der Ansprüche 10 bis 13, **dadurch gekennzeichnet, dass** der Bodensensor (1) während der Arbeitsfahrten von der Arbeitsmaschine in einer Geschwindigkeit von vorzugsweise maximal 15 km/h über den zu untersuchenden Untergrund bewegt wird, wobei die detektierte Beschaffenheit des Untergrunds, beispielsweise die Bodenart, unmittelbar zur Steuerung von im Heckbereich der Arbeitsmaschine montierten Bodenbearbeitungsgeräten verwendet wird.

15. Verfahren nach einem der Ansprüche 10 bis 14, **dadurch gekennzeichnet, dass** die Beschaffenheit des Untergrunds in einer ersten Arbeitsfahrt vorab, beispielsweise während der Aussaat, aufgenommen und in einer Datenbank gespeichert wird und in einer späteren Arbeitsfahrt zur Bodenbearbeitung eingesetzt wird.

## Claims

1. An agricultural work machine (7), in particular a tractor, comprising a ground sensor (1) for detecting the characteristics of a ground, wherein the ground sensor (1) includes at least one transmitter coil (2) and at least one, preferably four, receiver coil(s) (3), wherein the transmitter coil (2) is configured to generate an electromagnetic primary field and the receiver coil (3) is configured to receive the electromagnetic secondary field that is induced in the ground by the primary field, wherein the transmitter coil (2) and the receiver coil (3) are arranged in a housing (4) that includes a material that shields from electromagnetic radiation, **characterized in that** the ground sensor (1) is mounted on a front lifting mechanism of the work machine (7).

2. The agricultural work machine (7) according to Claim 1, **characterized in that** the housing (4) of the ground sensor includes an electrically nonconductive composite material, preferably a multilayer glass fiber composite material.

3. The agricultural work machine (7) according to Claim 1 or 2, **characterized in that** the housing (4) of the ground sensor includes a nonwoven (5) that shields from electromagnetic radiation, the nonwoven preferably being arranged in the whole housing wall apart from the area beneath the coils (2, 3) .

4. The agricultural work machine (7) according to Claim 3, **characterized in that** a grounding line that is electrically connected to the nonwoven (5) is provided for connecting the nonwoven (5) to an external grounding of a work machine.

5. The agricultural work machine (7) according to one of Claims 1 to 4, **characterized in that** at least one distance sensor for determining the distance of the device from the ground is provided on the ground sensor.

6. The agricultural work machine (7) according to one of Claims 1 to 5, **characterized in that** at least one inclination sensor for determining the inclination of the device relative to the ground is provided on the ground sensor.

7. The agricultural work machine (7) according to one of Claims 1 to 6, **characterized in that** at least one localization module, preferably a GPS module, is provided on the ground sensor.

8. The agricultural work machine (7) according to one of Claims 1 to 7, **characterized in that** a processing unit (8) is provided for converting the signals received by the receiver coil (3) into electrical conductivity values and ground parameters such as density, moisture, and soil type.

9. The agricultural work machine (7) according to one of Claims 1 to 8, **characterized in that** control outputs (10) for controlling external soil-working devices are provided on the work machine (7), wherein the control outputs (10) are preferably arranged in the rear area of the work machine so that direct control of soil-working devices arranged in the rear area of the work machine is made possible based on the ground characteristics detected by the ground sensor (1) during a work trip.

10. A method for operating an agricultural work machine according to one of Claims 1 to 9, **characterized in that** the ground parameters of the section of ground to be worked at that moment are detected during a work trip of the work machine.

11. The method according to Claim 10, **characterized in that** for calibrating the ground sensor (1), initially a one-time static measurement without the work machine is carried out, and based on the background noise detected in the static measurement, a static offset is determined, and the signals detected during the work trips are subsequently corrected by this static offset.

12. The method according to Claim 10 or 11, **characterized in that** the signals detected during the work trips are filtered by an adaptive low pass filter, wherein the parameters of the low pass filter are adaptable during the work trip.

13. The method according to Claim 12, **characterized in that** the cutoff frequency of the low pass filter is set in an initialization phase, and during a work trip is adjusted upon exceeding a limit value of the amplitude of the measured signals.

14. The method according to one of Claims 10 to 13, **characterized in that** during the work trips, the ground sensor (1) is moved across the ground to be examined by the work machine at a speed of preferably 15 km/h maximum, wherein the detected characteristics of the ground, for example the soil type, are directly used for controlling soil-working devices mounted in the rear area of the work machine.

15. The method according to one of Claims 10 to 14, **characterized in that** the characteristics of the ground are recorded in advance in a first work trip, for example during the sowing, and stored in a database and used in a subsequent work trip for the soil working.

## Revendications

1. Machine agricole de travail (7), en particulier un tracteur, comprenant un capteur de sol (1) pour détecter la nature d'un terrain, dans laquelle le capteur de sol (1) comprend au moins une bobine émettrice (2) et au moins une, de préférence quatre, bobine(s) réceptrice(s) (3), dans laquelle la bobine émettrice (2) est agencée pour produire un champ primaire électromagnétique et la bobine réceptrice (3) est agencée pour recevoir le champ secondaire électromagnétique induit dans le terrain par le champ primaire, dans laquelle la bobine émettrice (2) et la bobine réceptrice (3) sont disposées dans un boîtier (4) comprenant un matériau de blindage contre les rayons électromagnétiques, **caractérisée en ce que** le capteur de sol (1) est monté sur un dispositif de levage avant de la machine de travail (7).

2. Machine agricole de travail (7) selon la revendication 1, **caractérisée en ce que** le boîtier (4) du capteur de sol comprend un matériau composite non conducteur électriquement, de préférence un matériau composite de fibres de verre multicouche.

3. Machine agricole de travail (7) selon la revendication 1 ou 2, **caractérisée en ce que** le boîtier (4) du capteur de sol comprend un non-tissé (5) de blindage contre les rayons électromagnétiques qui est disposé de préférence dans toute la paroi de boîtier à l'exception de la zone au-dessous des bobines (2, 3).

4. Machine agricole de travail (7) selon la revendication 3, **caractérisée en ce qu'**un conducteur de mise à la terre, relié électriquement au non-tissé (5), est prévu pour relier le non-tissé (5) à la mise à la terre externe d'une machine de travail.

5. Machine agricole de travail (7) selon l'une quelconque des revendications 1 à 4, **caractérisée en ce que** sur le capteur de sol, au moins un capteur de distance est prévu pour déterminer la distance entre le dispositif et le terrain.

6. Machine agricole de travail (7) selon l'une quelconque des revendications 1 à 5, **caractérisée en ce que** sur le capteur de sol, au moins un capteur d'inclinaison est prévu pour déterminer l'inclinaison du dispositif par rapport au terrain.

7. Machine agricole de travail (7) selon l'une quelconque des revendications 1 à 6, **caractérisée en ce que** sur le capteur de sol, au moins un module de localisation, de préférence un module GPS, est prévu.

8. Machine agricole de travail (7) selon l'une quelconque des revendications 1 à 7, **caractérisée en ce qu'**une unité de calcul (8) est prévue pour convertir les signaux reçus par la bobine réceptrice (3) en valeurs de conductibilité électrique et en paramètres de sol, par exemple densité, humidité et type de sol.

9. Machine agricole de travail selon l'une quelconque des revendications 1 à 8, **caractérisée en ce que** sur la machine de travail (7) des sorties de commande (10) sont prévues pour piloter des appareils de travail du sol externes, dans laquelle les sorties de commande (10) sont disposées de préférence à l'arrière de la machine de travail de façon à permettre un pilotage direct des appareils pour le travail du sol disposés à l'arrière de la machine sur la base de la nature du sol détectée par le capteur de sol (1) pendant un trajet de travail.

10. Procédé de fonctionnement d'une machine agricole de travail selon l'une quelconque des revendications 1 à 9, **caractérisé en ce que** pendant un trajet de travail de la machine de travail, les paramètres de sol de la partie de sol à travailler actuellement sont détectés.

11. Procédé selon la revendication 10, **caractérisé en ce que** pour le calibrage du capteur de sol (1), d'abord une mesure statique unique est effectuée sans la machine de travail, et sur la base du bruit de fond détecté dans la mesure statique, un décalage statique est déterminé, et ensuite, les signaux détectés pendant les trajets de travail sont corrigés par ce décalage statique.

12. Procédé selon la revendication 10 ou 11, **caractérisé en ce que** les signaux détectés pendant les trajets de travail sont filtrés par un filtre passe-bas adaptatif, les paramètres du filtre passe-bas pouvant être adaptés pendant le trajet de travail.

13. Procédé selon la revendication 12, **caractérisé en ce que** la fréquence de coupure du filtre passe-bas est fixée dans une phase d'initialisation et est adaptée pendant un trajet de travail en cas de dépassement d'une valeur limite de l'amplitude des signaux mesurés.

14. Procédé selon l'une quelconque des revendications 10 à 13, **caractérisé en ce que** le capteur de sol (1) est déplacé sur le terrain à examiner pendant les trajets de travail de la machine de travail à une vitesse de préférence d'un maximum de 15 km/h, la nature détectée du terrain, par exemple le type de sol, étant utilisée directement pour commander des appareils de travail du sol montés à l'arrière de la machine de travail.

15. Procédé selon l'une quelconque des revendications 10 à 14, **caractérisé en ce que** la nature du terrain est enregistrée au préalable lors d'un premier trajet de travail, par exemple pendant l'ensemencement, et stockée dans une base de données et utilisée lors d'un trajet de travail ultérieur pour le travail du sol.
